# EUROPEAN PATENT SPECIFICATION

(11) **EP 3 841 992 B1**
(45) Date of publication and mention of the grant of the patent: **26.06.2024**
(21) Application number: 20215386.2
(22) Date of filing: 18.12.2020
(51) Int. Cl.: A61B 17/88, A61B 17/70

(54) **TRUMPET EXPANDABLE VERTEBRAL DEVICE**
ERWEITERBARE TROMPETENWIRBELVORRICHTUNG
DISPOSITIF VERTÉBRAL EXTENSIBLE EN TROMPETTE

(30) Priority: 18.12.2019 TW 108146387
(43) Date of publication of application: 30.06.2021
(73) Proprietor: BioLife Medical Device Inc., Hsinchu City 300 (TW)
(72) Inventor: CHEN, Li-Sen, Hsinchu City 300 (TW); CHAO, Che-Yang, Hsinchu City 300 (TW); KAO, Li-Hwan, Hsinchu City 300 (TW)
(74) Representative: Viering, Jentschura & Partner mbB Patent- und Rechtsanwälte

(56) References cited:
- EP-A1- 3 219 273
- US-A1- 2005 070 911
- US-A1- 2015 374 507

## Description

### BACKGROUND OF THE INVENTION

### Field of Invention

The present invention relates to a trumpet expandable vertebral device; particularly, it relates to such a trumpet expandable vertebral device which, when inserted into a human body, can radially expand itself so as to assist restoring a vertebra to its original shape.

### Description of Related Art

When a human spine (e.g. cervical vertebrae, thoracic vertebrae, lumbar vertebrae, etc.) suffers a compression fracture, or collapses due to poor bone mass density, an expandable vertebral device which is inserted into the spine can help to expand the space to restore the spine to a better condition. US 7,846,206 B2, US 9,414,933 B2, and TW 1589266 disclose different types of expandable vertebral devices.

In comparison with the prior arts, the present invention provides a different mechanical structure to reduce the number of required components, whereby the manufacture and assembly of the vertebral device is much easier. EP 3 219 273 discloses EP 3 219 273 A1 discloses a trumpet expandable vertebral device, comprising: a center shaft having a front end and a rear end; a trumpet expandable part holding and surrounding the center shaft, wherein the trumpet expandable part includes: plural expandable plates, each expandable plate having a thickness which increases from the front end toward the rear end; a fixed part, for fixing a relative position of the trumpet expandable part with respect to the center shaft in an axial direction of the center shaft, but allowing the center shaft to rotate with respect to the trumpet expandable part within the fixed part in a radial direction of the center shaft; and a connection part for connecting the trumpet expandable plates with the fixed part; and a trumpet expanding ring mounted outside and surrounding the center shaft, wherein the trumpet expanding ring is located between the center shaft and the trumpet expandable part in the radial direction; wherein when the trumpet expanding ring moves toward the rear end relatively to the trumpet expandable part, the trumpet expandable plates are expanded to restore the height and shape of a vertebra.

### SUMMARY OF THE INVENTION

According to the present invention, a trumpet expandable vertebral device as defined in claim 1 is provided. The dependent claims show some examples thereof.

In one embodiment, the center shaft has a main body and an exterior surface of the main body is threaded, and wherein an interior surface of the trumpet expanding ring is correspondingly threaded.

In one embodiment, the rear end of the center shaft is an operable end which is configured to be driven by a corresponding tool, such that the center shaft rotates within the fixed part and rotates relatively to the expandable plates.

In one embodiment, the operable end has a hexagonal shape.

In one embodiment, the trumpet expanding ring includes at least one protrusion which is configured to insert into a slit which is formed when the expandable plates are expanded, to be in contact with a side of at least one of the expandable plates.

In one embodiment, the center shaft includes a peripheral surface and the fixed part is mounted to press against the peripheral surface.

In one embodiment, the trumpet expandable vertebral device further comprises a nut which is connected to the front end of the center shaft.

In one embodiment, the center shaft comprises a hollow interior and is provided with an inlet and at least one outlet for the injection of a filler into a human body.

In one embodiment, the center shaft is provided with at least one outlet on a radial surface of the rotatable center shaft.

In one embodiment, each of the expandable plates has a width D, and the connection part is connected with each expandable plate by a width d, and d<(1/2)D.

The objectives, technical details, features, and effects of the present invention will be better understood with regard to the detailed description of the embodiments below, with reference to the attached drawings.

### BRIEF DESCRIPTION OF THE DRAWINGS

Fig. 1 shows a trumpet expandable vertebral device according to one embodiment of the present invention, in expanded state.
Fig. 2 shows the trumpet expandable vertebral device in Fig. 1, in disassembled state.
Fig. 3 shows the trumpet expandable vertebral device in Fig. 1, in closed state.
Fig. 4 shows a trumpet expandable vertebral device according to another embodiment of the present invention, in expanded state.
Fig. 5 shows the trumpet expandable vertebral device in Fig. 4, in disassembled state.

### DESCRIPTION OF THE PREFERRED EMBODIMENTS

The drawings as referred to throughout the description of the present invention are for illustration only, to show the interrelations between the components, but not drawn according to actual scale of shapes and sizes.

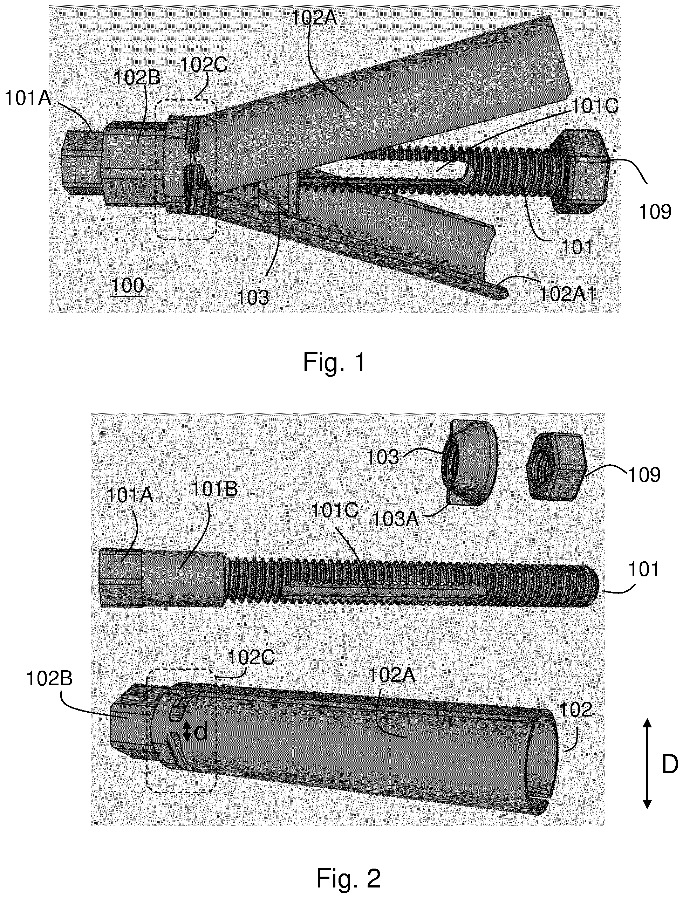

Please refer to Figs. 1-3, which show a trumpet expandable vertebral device according to an embodiment of the present invention (i.e. trumpet expandable vertebral device 100), in its expanded state (Fig. 1), disassembled state (Fig. 2) and closed state, respectively. The right side, in the drawings, of the trumpet expandable vertebral device 100 is the front end and the left side of the trumpet expandable vertebral device 100 is the rear end. When an surgical operation is performed to insert the trumpet expandable vertebral device 100 into a human body, the right side enters the human body first.

The trumpet expandable vertebral device 100, according to the present invention, includes a center shaft 101, a trumpet expandable part 102 and a trumpet expanding ring 103. The trumpet expandable part 102 and the trumpet expanding ring 103 are mounted outside and surrounding the center shaft 101. By the term "mounted", it means that the trumpet expandable part 102 and the trumpet expanding ring 103 surround and hold the center shaft 101, but it is not necessary for the trumpet expandable part 102 and the trumpet expanding ring 103 to be fixed to the center shaft 101. In a radial direction of the center shaft 101, the trumpet expanding ring 103 is located between the center shaft 101 and the trumpet expandable part 102. The center shaft 101 has a main body and an exterior surface of the main body is threaded, and an interior surface of the trumpet expanding ring 103 is correspondingly threaded. The rear end (left side) of the center shaft 101 is an operable end 101A; when an surgical operation is performed and the operable end 101A is activated to rotate, the center shaft 101 is driven to rotate within the trumpet expanding ring 103 and relatively to the trumpet expanding ring 103, whereby the center shaft 101 moves relatively to the trumpet expanding ring 103 along an axial direction of the center shaft 101 (that is, whereby the trumpet expanding ring 103 moves relatively to the center shaft 101 along the axial direction of the center shaft 101). In the embodiment as shown in Fig. 2, the operable end 101A has a hexagonal shape, and can be driven by a tool having a corresponding shape. However, the present invention can be embodied in many other ways, as long as the operable end 101A can be driven by a corresponding tool.

The trumpet expandable part 102 includes expandable plates 102A, a fixed part 102B and a connection part 102C. There are plural expandable plates 102A; each expandable plate 102A has a thickness 102A1 which increases from the front end (right side of the figure) toward the rear end (left side of the figure) . This thickness variation is an important feature of the present invention and will be explained in more detail later below. In the shown embodiment, the expandable plates 102A have a rectangular shape in a projection surface which is parallel to the axial direction of the center shaft 101 and have an arc shape in another projection surface which is parallel to the radial direction of the center shaft 101, but this is only one example and the shape of the expandable plates 102A can be embodied in other forms. The fixed part 102B is configured to fix a relative position of the trumpet expandable part 102 with respect to the center shaft 101 in the axial direction of the center shaft 101, that is, so that the positional relationship between the trumpet expandable part 102 and the center shaft 101 is held stable. The relative position between the trumpet expandable part 102 and the center shaft 101 can be fixed by any feasible ways, and what is shown by the embodiment is one example. In this embodiment as shown in the drawings, the center shaft 101 includes a peripheral surface 101B and the fixed part 102B is mounted outside the peripheral surface 101B of the center shaft 101, to press against the operable end 101A. Thus, in the axial direction of the center shaft 101, the relative relationship between the trumpet expandable part 102 and the center shaft 101 is fixed relatively, while in the radial direction of the center shaft 101, the center shaft 101 is able to rotate within the fixed part 102B, relatively to the trumpet expandable part 102. The connection part 102C connects the expandable plates 102A with the fixed part 102B.

When an operator, such as a surgeon, drives the center shaft 101 to rotate relatively to the trumpet expanding ring 103 (which also is to rotate relatively to the trumpet expandable part 102), whereby the center shaft 101 move within and relatively to the fixed part 103 toward the right side, the trumpet expanding ring 103 can be driven toward the left side relatively to the trumpet expandable part 102. Because the thickness 102A1 of the expandable plate 102A is varied, such as constantly increasing from the right side of the figure toward the left side of the figure, when the trumpet expanding ring 103 is driven toward the left side relatively to the trumpet expandable part 102, the expandable plates 102A are expanded, achieving the function of restoring the height and shape of the vertebrae.

Please refer to Fig. 2. In one preferred embodiment, the trumpet expanding ring 103 includes at least one protrusion 103A, and more preferably, includes protrusions 103A having a number equal to the number of the expandable plates 102A. The protrusion 103A is configured to be inserted into a slit which is formed between the lateral edges of the expandable plates 102A when they are expanded, to be in contact with a side edge of a corresponding expandable plate 102A. Thus, when the expandable plates 102A tend to compress due to a resistance coming from human body, because the protrusion 103A is in contact with or limited by the edges of the expandable plate 102A, the trumpet expanding ring 103 can be held in the desired position and held not to be pushed backward according to the axial direction of the center shaft 101.

In one preferred embodiment, the center shaft 101 comprises a hollow interior and is provided with an inlet and at least one outlet for the injection of a filler into a human body during a surgical operation, so that when the trumpet expandable vertebral device 100 is inserted into the human body, the filler such as bone cement or bone graft can be injected from the inlet, through the hollow interior, to outflow from the outlet into the vertebrae. In one embodiment, the filler is injected form the inlet at the left side (at the operable end 101A) of the center shaft 101, through the hollow interior of the center shaft 101, to outflow out from the outlet at the right side of the center shaft 101. Referring to Figs. 1 and 2, in a more preferable embodiment, the center shaft 101 is further provided with outlets (e.g. 101C) on its radial surface to multiply the channels for releasing the fillers, so that the filler injected form the inlet at the left side of the center shaft 101, in addition to flowing through the hollow interior of the center shaft 101 and flowing out from the outlet at the right side of the center shaft 101, also flows out in the radial direction from the radial outlets provided on the radial surface of the center shaft 101. The number, size and shape of the outlets 101C are shown as an example, not for limiting the scope of the present invention.

Please refer to Fig. 2, in one preferred embodiment, each expandable plate 102A has a width D (i.e. the perimeter of the expandable plate 102A in the radial surface), and the connection part 102C is connected with each expandable plate 102A by a width d, and d<D, to reduce the required force for expanding the expandable plate 102A. In a more preferable embodiment, d<(1/2)D.

In one preferred embodiment, a nut 109 is fitted to or is fixed to the right side of the center shaft 101, to prevent the trumpet expanding ring 103 from detaching from the center shaft 101. However, if this is not a concern, the nut 109 can be omitted.

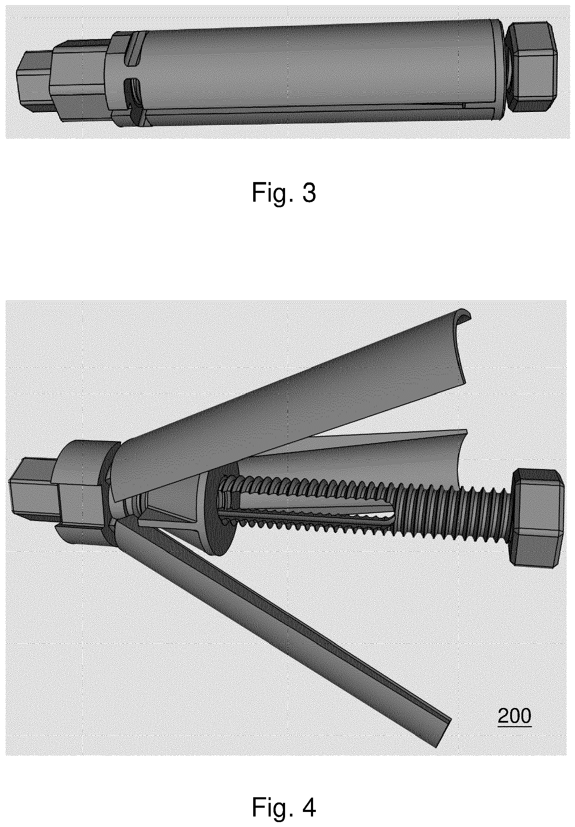

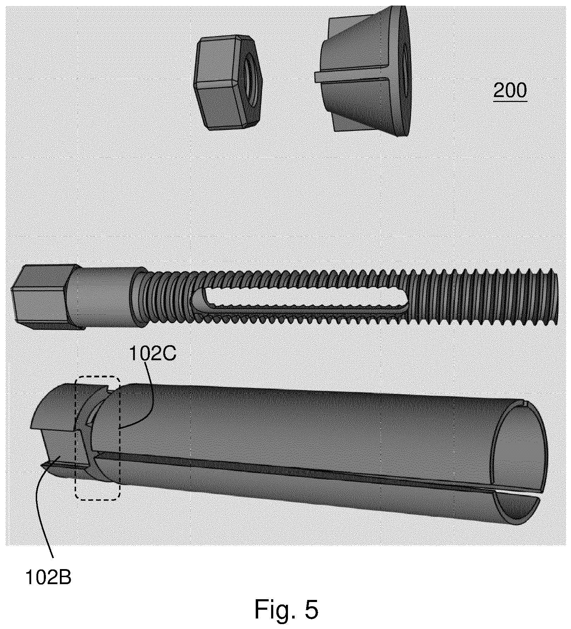

Please refer to Figs. 4-5, which show a trumpet expandable vertebral device according to another embodiment of the present invention, in expanded state and in disassembled state, respectively. This embodiment shows that: the number of the expandable plates is not limited to two, but can be a number more than two; so is the number of the protrusions 103A of the trumpet expanding ring 103. Also, please refer to Fig. 5, the shapes and structures of the fixed part 102B and the connection part 102C are not limited to the shapes and structures as shown in Figs. 1-3, but can be modified as desired; the scope of the present invention is fulfilled as long as the fixed part 102B can assist to fix the relative relationship between the trumpet expandable part 102 and center shaft 101 in the axial direction of the center shaft 101, and the connection part 102C allows the expandable plate 102A to expand.

## Claims

1. A trumpet expandable vertebral device (100), comprising:
a center shaft (101) having a front end and a rear end (101A);
a trumpet expandable part (102) holding and surrounding the center shaft, wherein the trumpet expandable part (102) includes:
plural expandable plates (102A), each expandable plate having a thickness which increases from the front end toward the rear end;
a fixed part (102B), for fixing a relative position of the trumpet expandable part (102) with respect to the center shaft in an axial direction of the center shaft, but allowing the center shaft to rotate with respect to the trumpet expandable part (102) within the fixed part (102B) in a radial direction of the center shaft; and
a connection part (102C) for connecting the trumpet expandable plates with the fixed part; and a trumpet expanding ring (103) mounted outside and surrounding the center shaft, wherein the trumpet expanding ring (103) is located between the center shaft and an internal surface of each expandable plate in the radial direction, and the trumpet expanding ring (103) is in contact with the center shaft and in contact with the internal surface of each expandable plate;
wherein when the trumpet expanding ring (103) moves toward the rear end relatively to the trumpet expandable part (102), the trumpet expandable plates are expanded to restore the height and shape of a vertebra, and wherein the trumpet expanding ring (103) is able to stop at anyone of plural positions relative to the central shaft as the trumpet expanding ring (103) moves toward the rear end relatively to the trumpet expandable part (102) and still steadily fixed at that position to be in contact and in cooperation with the thickness of the expandable plate at that position whereby the trumpet expandable part (102) is able to expand by anyone of plural angle degrees and still steadily fixed at that angle degree.

2. The trumpet expandable vertebral device of claim 1, wherein the center shaft (101) has a main body and an exterior surface of the main body is threaded, and wherein an interior surface of the trumpet expanding ring (103) is correspondingly threaded.

3. The trumpet expandable vertebral device of claim 1, wherein the rear end of the center shaft (101) is an operable end (101A) which is configured to be driven by a corresponding tool, such that the center shaft (101) rotates within the fixed part (102B) and rotates relatively to the expandable plates (102A).

4. The trumpet expandable vertebral device of claim 3, wherein the operable end (101A) has a hexagonal shape.

5. The trumpet expandable vertebral device of claim 1, wherein the trumpet expanding ring (103) includes at least one protrusion (103A) which is configured to insert into a slit which is formed when the expandable plates (102A) are expanded, to be in contact with a side of at least one of the expandable plates (102A).

6. The trumpet expandable vertebral device of claim 1, wherein the center shaft (101) includes a peripheral surface and the fixed part (102B) is mounted outside the peripheral surface.

7. The trumpet expandable vertebral device of claim 1, further comprising a nut (109) which is connected to the front end of the center shaft (101).

8. The trumpet expandable vertebral device of claim 1, wherein the center shaft (101) comprises a hollow interior and is provided with an inlet and at least one outlet for the injection of a filler into a human body.

9. The trumpet expandable vertebral device of claim 8, wherein the center shaft (101) is provided with at least one outlet on a radial surface of the rotatable center shaft (101).

10. The trumpet expandable vertebral device of claim 1, wherein each of the expandable plates (102A) has a width D, and the connection part (102C) is connected with each expandable plate (102A) by a width d, and d<(1/2)D.

## Patentansprüche

1. Eine trompetenartig spreizbare Wirbelvorrichtung (100), umfassend:
einen Mittelschaft (101) mit einem vorderen Ende und einem hinteren Ende (101A);
ein trompetenartig spreizbares Teil (102), das den Mittelschaft hält und umgibt, wobei das trompetenartig spreizbare Teil (102) aufweist:
mehrere spreizbare Platten (102A), wobei jede spreizbare Platte eine Dicke aufweist, die vom vorderen Ende zum hinteren Ende hin zunimmt;
ein feststehendes Teil (102B) zum Fixieren einer relativen Position des trompetenartig spreizbaren Teils (102) in Bezug auf den Mittelschaft in einer axialen Richtung des Mittelschafts, wobei jedoch der Mittelschaft in Bezug auf das trompetenartig spreizbare Teil (102) innerhalb des feststehenden Teils (102B) in einer radialen Richtung des Mittelschafts rotieren kann; und
ein Verbindungsteil (102C) zum Verbinden der trompetenartig spreizbaren Platten mit dem feststehenden Teil; und
einen Trompetenspreizring (103), der außerhalb des Mittelschafts angebracht ist und diesen umgibt, wobei der Trompetenspreizring (103) zwischen dem Mittelschaft und einer Innenfläche jeder spreizbaren Platte in der radialen Richtung angeordnet ist und der Trompetenspreizring (103) in Kontakt mit dem Mittelschaft und in Kontakt mit der Innenfläche jeder spreizbaren Platte ist;
wobei, wenn sich der Trompetenspreizring (103) in Richtung des hinteren Endes relativ zu dem trompetenartig spreizbaren Teil (102) bewegt, die trompetenartig spreizbaren Platten gespreizt werden, um die Höhe und Form eines Wirbels wiederherzustellen, und wobei der Trompetenspreizring (103) in der Lage ist, an irgendeiner von mehreren Positionen relativ zu dem zentralen Schaft zu stoppen, wenn sich der Trompetenspreizring (103) in Richtung des hinteren Endes relativ zu dem trompetenartig spreizbaren Teil (102) bewegt und weiterhin dauerhaft an dieser Position fixiert ist, um in Kontakt und in Zusammenwirkung mit der Dicke der spreizbaren Platte an dieser Position zu sein, wodurch das trompetenartig spreizbare Teil (102) in der Lage ist, sich um irgendeinen von mehreren Winkelgraden zu spreizen und weiterhin dauerhaft bei diesem Winkelgrad fixiert ist.

2. Die trompetenartig spreizbare Wirbelvorrichtung nach Anspruch 1, wobei der Mittelschaft (101) einen Hauptkörper aufweist und eine Außenfläche des Hauptkörpers mit einem Gewinde versehen ist, und wobei eine Innenfläche des Trompetenspreizrings (103) entsprechend mit einem Gewinde versehen ist.

3. Die trompetenartig spreizbare Wirbelvorrichtung nach Anspruch 1, wobei das hintere Ende des Mittelschafts (101) ein betätigbares Ende (101A) ist, das eingerichtet ist, um von einem entsprechenden Gerät angetrieben zu werden, so dass sich der Mittelschaft (101) innerhalb des feststehenden Teils (102B) dreht und sich relativ zu den spreizbaren Platten (102A) dreht.

4. Die trompetenartig spreizbare Wirbelvorrichtung nach Anspruch 3, wobei das betätigbare Ende (101A) eine sechseckige Form aufweist.

5. Die trompetenartig spreizbare Wirbelvorrichtung nach Anspruch 1, wobei der Trompetenspreizring (103) mindestens einen Vorsprung (103A) aufweist, der eingerichtet ist, um in einen Schlitz eingeführt zu werden, der gebildet wird, wenn die spreizbaren Platten (102A) gespreizt werden, um in Kontakt mit einer Seite von mindestens einer der spreizbaren Platten (102A) zu sein.

6. Die trompetenartig spreizbare Wirbelvorrichtung nach Anspruch 1, wobei der Mittelschaft (101) eine Umfangsfläche aufweist und das festehende Teil (102B) außerhalb der Umfangsfläche angebracht ist.

7. Die trompetenartig spreizbare Wirbelvorrichtung nach Anspruch 1, ferner aufweisend eine Mutter (109), die mit dem vorderen Ende des Mittelschafts (101) verbunden ist.

8. Die trompetenartig spreizbare Wirbelvorrichtung nach Anspruch 1, wobei der Mittelschaft (101) einen hohlen Innenraum aufweist und mit einem Einlass und mindestens einem Auslass für die Injektion eines Füllstoffs in einen menschlichen Körper versehen ist.

9. Die trompetenartig spreizbare Wirbelvorrichtung nach Anspruch 8, wobei der Mittelschaft (101) mit mindestens einem Auslass an einer radialen Oberfläche des drehbaren Mittelschafts (101) versehen ist.

10. Die trompetenartig spreizbare Wirbelvorrichtung nach Anspruch 1, wobei jede der spreizbaren Platten (102A) eine Breite D aufweist und das Verbindungsteil (102C) mit jeder spreizbaren Platte (102A) durch eine Breite d verbunden ist und d< (1/2) D.

## Revendications

1. Dispositif vertébral à trompette expansible (100), comprenant :
un arbre central (101) ayant une extrémité avant et une extrémité arrière (101A) ;
une partie expansible de trompette (102) maintenant et entourant l'arbre central, la partie expansible de trompette (102) comprenant :
plusieurs plaques expansibles (102A), chaque plaque expansible ayant une épaisseur qui augmente de l'extrémité avant vers l'extrémité arrière,
une partie fixe (102B), pour fixer une position relative de la partie expansible de trompette (102) par rapport à l'arbre central dans une direction axiale de l'arbre central, tout en permettant à l'arbre central de tourner par rapport à la partie expansible de trompette (102) dans la partie fixe (102B) dans une direction radiale de l'arbre central ; et
une partie de connexion (102C) pour connecter les plaques expansibles de trompette à la partie fixe ; et
un anneau d'expansion de trompette (103) monté à l'extérieur et entourant l'arbre central, dans lequel l'anneau d'expansion de trompette (103) est situé entre l'arbre central et une surface interne de chaque plaque expansible dans la direction radiale, et l'anneau d'expansion de trompette (103) est en contact avec l'arbre central et en contact avec la surface interne de chaque plaque expansible ;
dans lequel, lorsque l'anneau d'expansion de trompette (103) se déplace vers l'extrémité arrière par rapport à la partie expansible de trompette (102), les plaques expansibles de trompette sont expansées pour restaurer la hauteur et la forme d'une vertèbre, et dans lequel l'anneau d'expansion de trompette (103) peut s'arrêter à n'importe laquelle de plusieurs positions par rapport à l'arbre central lorsque l'anneau d'expansion de trompette (103) se déplace vers l'extrémité arrière par rapport à la partie expansible de trompette (102) et reste fixé de manière stable à cette position pour être en contact et en coopération avec l'épaisseur de la plaque expansible à cette position, la partie expansible de trompette (102) pouvant ainsi s'étendre de n'importe lequel de plusieurs degrés d'angle et restant fixée de manière stable à ce degré d'angle.

2. Dispositif vertébral à trompette expansible selon la revendication 1, dans lequel l'arbre central (101) a un corps principal et une surface extérieure du corps principal est filetée, et dans lequel une surface intérieure de l'anneau expansible de trompette (103) est filetée de manière correspondante.

3. Dispositif vertébral à trompette expansible selon la revendication 1, dans lequel l'extrémité arrière de l'arbre central (101) est une extrémité opérationnelle (101A) qui est configurée pour être entraînée par un outil correspondant, de sorte que l'arbre central (101) tourne à l'intérieur de la partie fixe (102B) et tourne par rapport aux plaques expansibles (102A).

4. Dispositif vertébral à trompette expansible selon la revendication 3, dans lequel l'extrémité opérationnelle (101A) a une forme hexagonale.

5. Dispositif vertébral à trompette expansible selon la revendication 1, dans lequel l'anneau expansible de trompette (103) comprend au moins une saillie (103A) qui est configurée pour s'insérer dans une fente qui est formée lorsque les plaques expansibles (102A) sont expansées, pour être en contact avec un côté d'au moins une des plaques expansibles (102A) .

6. Dispositif vertébral à trompette expansible selon la revendication 1, dans lequel l'arbre central (101) comprend une surface périphérique et la partie fixe (102B) est montée à l'extérieur de la surface périphérique.

7. Dispositif vertébral à trompette expansible selon la revendication 1, comprenant en outre un écrou (109) qui est connecté à l'extrémité avant de l'arbre central (101).

8. Dispositif vertébral à trompette expansible selon la revendication 1, dans lequel l'arbre central (101) comprend un intérieur creux et est pourvu d'une entrée et d'au moins une sortie pour l'injection d'un produit de remplissage dans un corps humain.

9. Dispositif vertébral à trompette expansible selon la revendication 8, dans lequel l'arbre central (101) est pourvu d'au moins une sortie sur une surface radiale de l'arbre central rotatif (101).

10. Dispositif vertébral à trompette expansible selon la revendication 1, dans lequel chacune des plaques expansibles (102A) a une largeur D, et la partie de connexion (102C) est connectée à chaque plaque expansible (102A) par une largeur d, et d < (1/2) D.
